# EUROPEAN PATENT APPLICATION

(11) **EP 2 153 823 A1**
(43) Date of publication of application: **17.02.2010**
(21) Application number: 09153738.1
(22) Date of filing: 26.02.2009
(51) Int. Cl.: A61K 9/20

(54) **Double-layered zaleplon-containing tablet**

(30) Priority: 13.08.2008 TW 97130784
(71) Applicant: Orient Pharma Co., Ltd., Yunlin County Dounan Township, Taiwan (CN)
(72) Inventor: KE, Wen-Ting, Longtan Township (TW); LIN, Tse-Ching, Yingge Township (TW)
(74) Representative: Viering, Jentschura & Partner

(57) **Abstract**

Disclosed is a double-layered tablet containing zaleplon. The tablet includes an instant release layer and a delayed release layer. The instant release layer contains zaleplon, lactose, maltose, microcrystalline cellulose, sodium lauryl sulfate, and at least one excipient. The delayed release layer contains zaleplon, maltose, microcrystalline cellulose, a polymer, and at least one excipient. A dissolution curve is obtained for the double-layered zaleplon-contained tablet by using a 0.1 N hydrochloric acid buffer and pH6.8 phosphoric acid buffer contained in stirring devices having rotatable blades with stirring speeds of 50-200RPM, and the release of zaleplon is controlled in such a way to follow the dissolution curve, so as to provide the advantages of improving sleep quality, enhancing safety of medicine use, being convenient to use, and offering flexible dosing.

## Description

### FIELD OF THE INVENTION

The present invention relates to a double-layered tablet that contains zaleplon, and in particular to a tablet that comprises a combination of an instant release layer and a delayed release layer to provide an effect of improving sleep quality and is applicable to all sorts of sleeping disorders or the likes.

### BACKGROUND OF THE INVENTION

Sleep is a must to human beings and is considered as important as food and work. As a metal part that undergoes fatigue and deformation must be re-strengthened, human beings, who are not as strong as metals, must also take sleeps in order to remove tiredness of the body. This is the efficacy of sleep. With the development of modern technology, the modern people are living with a much increased pace, which, together with economic recessions that often occur in the human society and the severe job and market competition so caused, the modern people bear, more or less, psychological tension and job pressure and have to suffer disordered living, chaos of biological clock by for example sleeping late and waking up early just for making a success in job competition or passing an examination. As a result, problems, such as hard to fall asleep, bad sleeping, may occur, leading to next-day headache, metal dejection, and delay of response. It is even worse that such a situation, when persistent for a sufficiently long time, may adversely affect the metabolism and the normal functions of the immune system of human body. It is said that do not allow the tiredness of today to cause karoshi for tomorrow. Thus, to improve the sleep quality is now an issue that the modern people must pay careful attention to.

Hypnotics are an effective medicine commonly taken to treat insomnia. The hypnotics are generally classified as fugitive type and persistent type. The fugitive type hypnotics take effect in a very short period and usually serve to induce sleep of a medicine taker, meaning they are effective in the period of getting into sleep. The persistent type hypnotics serve to prolong the sleep, meaning they are effective in the deep sleep period. The fugitive type hypnotics that are available in the market include Sonata that contains zaleplon and Stilnox that contains zolpidem. The fugitive type hypnotics, after being orally taken, will reach the maximum concentration in blood in about one hour. Consequently, a taker may quickly fall asleep. Further, since the half life of the medicine in human blood is short, the concentration of the medicine in blood is quickly reduced so that no side effect of headache will happen in the next day. However, the effective period of the medicine is around 4-6 hours, so that when the taker unexpectedly gets awake in the midnight, the sleep cannot continue. On the other hand, for the persistent type hypnotics, the most typical ones in the market are Lunesta containing eszopiclone and Ambien or Ambien CR that contains zolpidem. The persistent type hypnotics may reach the maximum concentration in blood in 2-6 hours after being orally taken. The half life of the persistent hypnotics in blood is relatively long and may often cause side effects after wake up, such as doze off, impairment of memory, and poor coordination of action. They are suitable for severe and long-term sleeping disorder patients.

In view of the above problems, the present invention aims to provide a feasible solution to solve the above problems.

### SUMMARY OF THE INVENTION

An objective of the present invention is to provide a double-layered zaleplon-contained tablet that is a combination of an instant release layer and a delayed release layer to provide an effect of improving sleep quality and thus enhance the practicability and improvement of the present invention.

Another objective of the present invention is to provide a double-layered zaleplon-contained tablet, which provides a coloring effect occurring when a colorant contained in the instant release layer, the delayed release layer, or the coating layer is brought into contact with liquid, so as to improve the safety of medicine and thus enhance the practicability and improvement of the present invention.

A further objective of the present invention is to provide a double-layered zaleplon-contained tablet, wherein by making the double-layered tablet in the form of a tablet, it can be convenient taken thereby enhancing the convenience of the present invention.

Yet a further objective of the present invention is to provide a double-layered zaleplon-contained tablet, wherein an indent line is provided on a surface of the double-layered tablet for half breaking of the tablet, so that a medicine taker can selectively take only half dose thereby providing flexible dosing and thus enhancing the variation of the present invention.

To realize the above objectives, the present invention provides a double-layered zaleplon-contained tablet, comprising an instant release layer and a delayed release layer. The instant release layer comprises zaleplon, lactose, maltose, microcrystalline cellulose, sodium lauryl sulfate, and at least one excipient. The delayed release layer comprises zaleplon, maltose, microcrystalline cellulose, a polymer, and at least one excipient. The tablet is covered by a coating layer that covers the instant release layer and the delayed release layer to form a double-layered tablet. The coating layer comprises hydroxypropyl methyl cellulose, talc, and polyethylene glycol. The instant release layer, the delayed release layer, and the coating layer may selectively comprise an colorant for identification purposes. A dissolution curve is obtained for the double-layered zaleplon-contained tablet by using a 0.1 N hydrochloric acid buffer and pH6.8 phosphoric acid buffer contained in string devices having rotatable blades with stirring speeds of 50-200RPM. The dissolution curve includes an instant release phase and a delayed release phase, in which the instant release phase has a maximum duration of 30 minutes (preferably 5-15 minutes), and the delayed release phase represents the remaining dissolution occurring after the instant release phase. Approximately 30-60% (preferably, 30-60%) of the total amount of zaleplon is released in the instant release phase. Approximately 90% of the total amount of zaleplon is released in a period of 4-8 hours (preferably, 4-6 hours). The release of the zaleplon hypnotic is controlled in such a way to follow the dissolution curve. As such, sleep quality can be improved, safety of using medicine can be enhanced, the use of the medicine is made convenient, and flexible dosing can be realized.

### BRIEF DESCRIPTION OF THE DRAWINGS

The present invention will be apparent to those skilled in the art by reading the following description of preferred embodiments thereof with reference to the drawings, in which:

Figure 1 is a perspective view of a first embodiment of the present invention;

Figure 2 is a perspective view of a second embodiment of the present invention;

Figure 3 is a perspective view of a third embodiment of the present invention;

Figure 4 is a perspective view of a fourth embodiment of the present invention;

Figure 5 illustrates a dissolution curve of an instant release layer in accordance with the present invention;

Figure 6 illustrates dissolution curves of a delayed release layer in accordance with the present invention; and

Figure 7 illustrates dissolution curves of embodiments of the present invention.

### DETAILED DESCRIPTION OF THE PREFERRED EMBODIMENTS

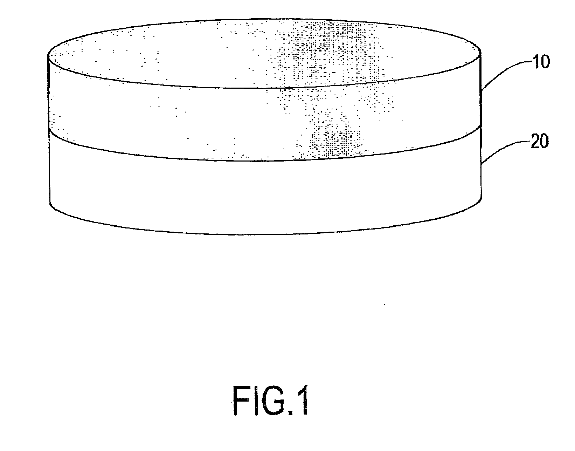

With reference to the drawings and in particular to Figure 1, which shows a perspective view of a medicine tablet in accordance with a first embodiment of the present invention, the medicine tablet of the present invention is a tablet containing zaleplon, of which the chemical name is N-(3-(3-cyanopyrazolo[1,5-a] pyrimidin-7-yl)phenyl)-N-ethylacetamide having a molecular formula C₁₇H₁₅N₅O and a molecular weight of 305.34. The double-layered tablet containing zaleplon comprises an instant release layer 10 and a delayed release layer 20.

The instant release layer 10 contains zaleplon, lactose, maltose, microcrystalline cellulose, sodium lauryl sulfate, and at least one excipient.

The delayed release layer 20 contains zaleplon, maltose, microcrystalline cellulose, a polymer, and at least one excipient.

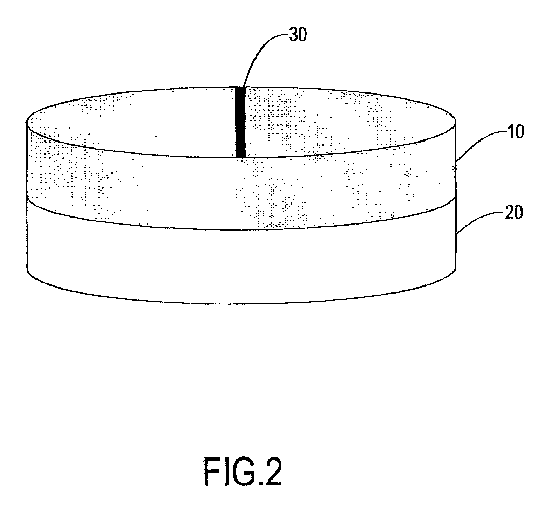

Referring to Figure 2, which shows a perspective view of a medicine tablet containing zaleplon in accordance with a second embodiment of the present invention, the tablet comprises an instant release layer 10 and a delayed release layer 20. The instant release layer 10 and the delayed release layer 20 respectively have the same ingredients as their counterparts of the first embodiment and no further description will be given. The feature of the second embodiment is that the instant release layer 10 and the delayed release layer 20 are processed by a tablet-making machine to form a double-layered table that has an indent line 30 on a surface thereof to ease half-breaking of the tablet by a user for flexible dosing.

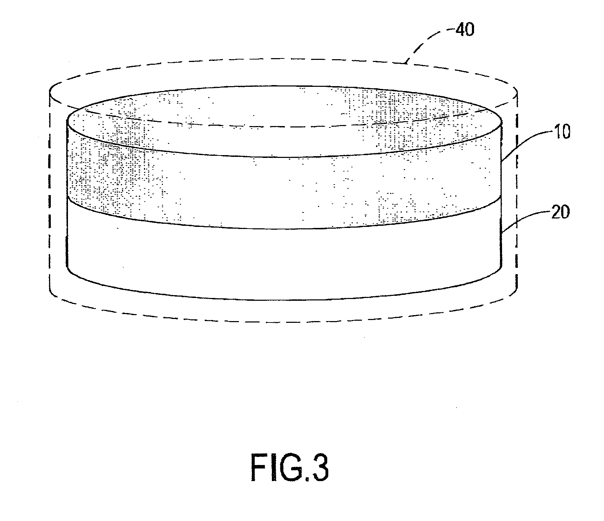

Referring to Figure 3, which shows a perspective view of a medicine tablet containing zaleplon in accordance with a third embodiment of the present invention, the tablet comprises an instant release layer 10, a delayed release layer 20, and a coating layer 40. The instant release layer 10 and the delayed release layer 20 respectively have the same ingredients as their counterparts of the previous embodiments and no further description will be given. The coating layer 40 contains hydroxypropyl methyl cellulose, talc, and polyethylene glycol (PEG) and, if desired, an colorant can be added (the colorant being selectively added to the instant release layer 10 and delayed release layer 20) to provide a coloring effect when contact with liquid for safety of medicine use. The colorant may be selected from any one of curcumins, riboflavins, tartrazine, quinoline yellow, sunset yellow FCF, alura red AC, patent blue V, carmines, azorubine, amaranth, erythrosine, indigotine, chlorophylls, green S, caramels, brown FK, brown HT, carotenes, lycopene, luteins, anthocyanidins, gardenia yellow, calcium carbonates, titanium dioxide, iron oxides, aluminum, silver, and gold.

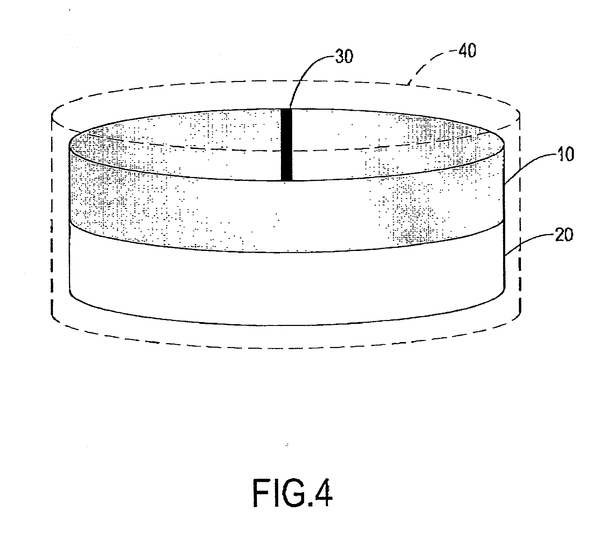

Referring to Figure 4, which shows a perspective view of a medicine tablet containing zaleplon in accordance with a fourth embodiment of the present invention, the tablet comprises an instant release layer 10, a delayed release layer 20, and a coating layer 40. The instant release layer 10, the delayed release layer 20, and the coating layer 40 respectively have the same ingredients as their counterparts of the first embodiment and no further description will be given. The feature of the fourth embodiment is that the instant release layer 10 and the delayed release layer 20 are processed by a tablet-making machine to form a double-layered table that has an indent line 30 on a surface thereof to ease half-breaking of the tablet by a user for flexible dosing.

The composition of an example of the instant release layer 10 in accordance with the present invention is given below:

| | Instant Release Layer | |
|---|---|---|
| Ingredient | mg/each tablet | percentage by weight (wt%) |
| zaleplon | 10.0 | 8.33 |
| lactose | 51.8 | 43.17 |
| maltose | 24.0 | 20.00 |
| microcrystalline cellulose | 24.0 | 20.00 |
| sodium lauryl sulfate | 1.2 | 1.00 |
| crospovidone | 6.0 | 5.00 |
| silicon dioxide | 2.4 | 2.00 |
| magnesium stearate | 0.6 | 0.50 |
| total | 120.0 | 100.00 |

The major ingredient zaleplon has the chemical name N-(3-(3-cyanopyrazolo[1,5-a] pyrimidin-7-yl)phenyl)-N-ethylacetamide that has a molecular formula C₁₇H₁₅N₅O and a molecular weight of 305.34

The ingredient sodium lauryl sulfate serves as a surfactant that provides excellent lubrication, enhances mechanical strength of the tablet, and facilitates compression, as well as collapsing and releasing of medicine. The ingredient crospovidone serves as a disintegrant, which can also be selected from any one of sodium starch glycolate, cross-carmellose sodium, low-substituted hydroxypropyl cellulose, microcrystalline cellulose, sodium lauryl sulfate, starch, pre-gelatinized starch, hydroxypropy starch, gum Arabic, tragacanth, karaya, pectin, agar, and locust bean gum. The ingredient silicon dioxide serves as a glidant, which can also be selected from talc or cornstarch. The ingredient magnesium stearate serves as a lubricant, which can also be selected from any one of calcium stearate, zinc stearate, stearic acid, hydrogenated vegetable oil, mineral oil, talc, or polyethylene glycol.

The instant release layer 10 functions to induce a taker of the tablet into sleep and can be made by direct compression of a mixture of zaleplon, lactose, maltose, microcrystalline cellulose, sodium lauryl sulfate, crospovidone, silicon dioxide, and magnesium stearate, or pelleting the mixture of zaleplon, lactose, maltose, microcrystalline cellulose, sodium lauryl sulfate, crospovidone, and silicon dioxide with water or solvents and then sieving and drying the pellets so obtained, followed by adding magnesium stearate and forming tablets with a tablet making machine. Apparently, other manufacturing processes that are known to those skilled in the art can also be used.

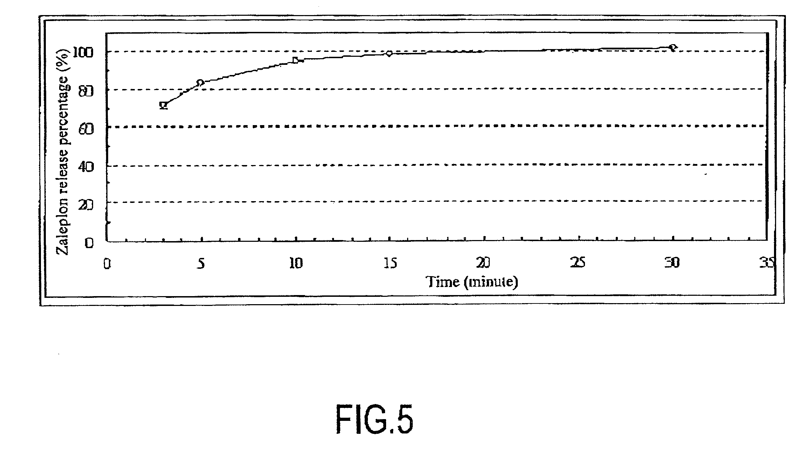

Referring to Figure 5, which shows a dissolution curve of the instant release layer 10 in accordance with the present invention, the dissolution curve is obtained by positioning the instant release layer 10 in 0.1N (normal concentration) hydrochloric acid buffer of 900ml at 37°C (for simulation of gastric juice) contained in a stirring device with a rotatable blade having a stirring speed of 75 RPM (revolution per minute). The dissolution curve indicates that the time period required to release 90% of the total amount of zaleplon is between 5 to 10 minutes and complete release can be achieved in 30 minutes.

The composition of an example of the delayed release layer 20 in accordance with the present invention is given below:

| | Delayed Release Layer | |
|---|---|---|
| | ZDT-022 (ZDT-20080513-1) | |
| Ingredient | mg/each tablet | percentage by weight (wt%) |
| zaleplon | 5.0 | 4.17 |
| maltose | 58.0 | 48.33 |
| microcrystalline cellulose | 24.0 | 20.00 |
| hydroxypropyl methyl cellulose | 30.0 | 25.00 |
| silicon dioxide | 2.4 | 2.00 |
| magnesium stearate | 0.6 | 0.50 |
| total | 120.0 | 100.00 |

The composition of another example of the delayed release layer 20 in accordance with the present invention is given below:

| Delayed Release Layer | | |
|---|---|---|
| ZDT-023 (ZDT-20080513-2) | | |
| Ingredient | mg/each tablet | percentage by weight (wt%) |
| zaleplon | 5.0 | 4.17 |
| maltose | 52.0 | 43.33 |
| microcrystalline cellulose | 24.0 | 20.00 |
| hydroxypropyl methyl cellulose | 36.0 | 30.00 |
| silicon dioxide | 2.4 | 2.00 |
| magnesium stearate | 0.6 | 0.50 |
| total | 120.0 | 100.00 |

The ingredient hydroxypropyl methyl cellulose is a hydrophilic polymer, which, when contacting aqueous liquids, may dissolve and expand to form a polymer network for controlling the release of zaleplon. This ingredient can also be selected from any one of hydroxymethyl cellulose, hydroxyethyl cellulose, hydroxypropyl cellulose, carboxymethyl cellulose, carboxymethylcellulose sodium, methylcellulose, ethylcellulose, gum Arabic, tragacanth, locust bean gum, guar, karaya, gelatin, pectin, casein, and polyethylene glycol. The ingredient silicon dioxide serves as a glidant, which can also be selected from talc or cornstarch. The ingredient magnesium stearate serves as a lubricant, which can also be selected from any one of calcium stearate, zinc stearate, stearic acid, hydrogenated vegetable oil, mineral oil, talc, or polyethylene glycol.

The delayed release layer 20 functions to maintain the sleep of the taker of the tablet and can be made by direct compression of a mixture of zaleplon, maltose, microcrystalline cellulose, hydroxypropyl methyl cellulose, silicon dioxide, and magnesium stearate, or pelleting the mixture of zaleplon, maltose, microcrystalline cellulose, hydroxypropyl methyl cellulose, and silicon dioxide with water or solvents and then sieving and drying the pellets so obtained, followed by adding magnesium stearate and forming tablets with a tablet making machine. Apparently, other manufacturing processes that are known to those skilled in the art can also be used.

Alternatively, medically acceptable organic acids may also be used to make the delayed release layer 20 for controlling pH value when the delayed release layer 20 is released in intestinal juice. The organic acid can be selected from lactic acid, citric acid, malic acid, maleic acid, fumaric acid, tartaric acid, succinic acid, or adipic acid, or acid salts thereof.

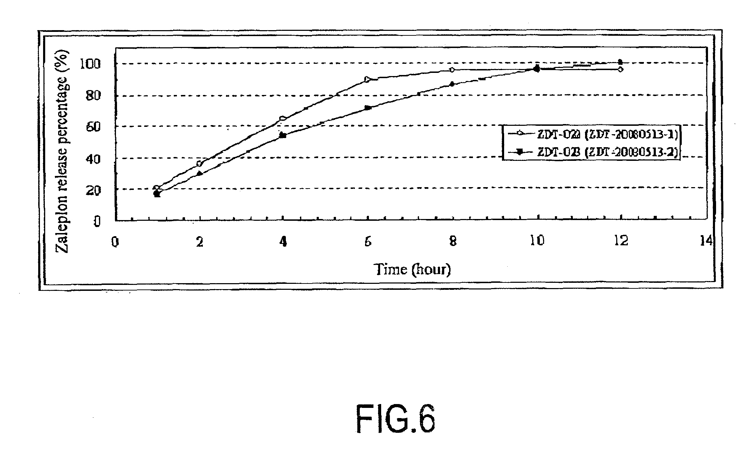

Referring to Figure 6, which shows dissolution curves of the delayed release layer 20 in accordance with the present invention, in which hollow circles indicate ZDT-022 and solid circles ZDT-023, the dissolution curves are obtained by positioning the instant release layer 10 in phosphoric acid buffer of 900ml at 37°C with a pH value of 6.8 (for simulation of intestinal juice) contained in a stirring device with a rotatable blade having a stirring speed of 100 RPM. The dissolution curves indicate that the time period required to release 90% of the total amount of zaleplon is between 5 to 7 hours.

The composition of an example of the coating layer 40 in accordance with the present invention is given below:

| Coating Layer | | |
|---|---|---|
| Ingredient | mg/each tablet | percentage by weight (wt%) |
| hydroxypropyl methyl cellulose | 8.4 | 70.00 |
| polyethylene glycol (PEG6000) | 1.8 | 15.00 |
| talc | 1.8 | 15.00 |
| total | 12.0 | 100.00 |

The coating layer 40 provides for moisture-proof, light-shielding, air isolation, enhancing medicine stability, and identification for improving safety of medicine use. The manufacturing of the coating layer 40 employs for example a coating machine or fluidized bed equipments to cover the instant release layer 10 and the delayed release layer 20 with a solution formed by mixing water or solvents with hydroxypropyl methyl cellulose, PEG6000, and talc, followed by drying and shaping. Alternatively, water or solvents are used to mix and pellet a mixture of hydroxypropyl methyl cellulose, PEG6000, and talc, followed by sieving and drying the pellets so obtained and then forming tablets together with the instant release layer 10 and the delayed release layer 20 in a tablet making machine. Of course, other manufacturing processes that are known to those skilled in the art can also be used. Further, the coating layer 40 can be added with an colorant to provide a coloring effect when contact with liquid for enhancing safety of medicine use. (Or alternatively and additionally, the colorant can be added in the instant release layer 10 and/or delayed release layer 20.) The colorant can be selected from any one of curcumins, riboflavins, tartrazine, quinoline yellow, sunset yellow FCF, allura red AC, patent blue V, carmines, azorubine, amaranth, erythrosine, indigotine, chlorophylls, green S, caramels, brown FK, brown HT, carotenes, lycopene, luteins, anthocyanidins, gardenia yellow, calcium carbonates, titanium dioxide, iron oxides, aluminum, silver, and gold. Further, the material for the coating layer can alternatively be selected from films that are available in the market, including Eudragit and Opadry.

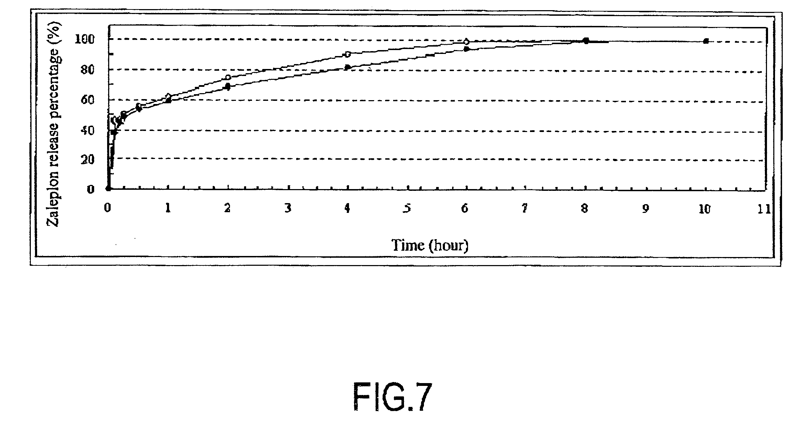

Referring to Figure 7, which shows dissolution curves of the instant embodiment of the present invention, in which hollow circles indicate a double-layered tablet formed by combining the instant release layer 10 and the delayed release layer 20 (ZDT-022) and solid circles indicate a double-layered tablet formed by combining the instant release layer 10 and the delayed release layer 20 (ZDT-023), the instant release layer 10 is separately combined with a delayed release layer 20 (ZDT-022) and another delayed release layer 20 (ZDT-023) by a tablet making machine to respectively form tablets, which are then separately deposited in 0.1 N hydrochloric acid buffer of 750ml at 37°C contained in a stirring device with a rotatable blade to be stirred for two hours at a stirring speed of 100 RPM and then put in phosphoric acid buffer of 1000ml at 37°C with a pH value of 6.8 contained in a stirring device with a rotatable blade to be stirred for eight hours at a stirring speed of 100 RPM. And the dissolution curves are measured. The dissolution curves so obtained each include an instant release phase and a delayed release phase. The instant release phase has a maximum duration of 30 minutes. The delayed release phase represents the remaining dissolution occurring after the instant release phase. Approximately 30-60% of the total amount of zaleplon is released in the instant release phase. Further, approximately 90% of the total amount of zaleplon is released in a period of 4-8 hours. For a double-layered zaleplon-contained tablet with a coating layer 40, the dissolution curves are similar to those shown in Figure 7. In addition, the coating layer 40 can be selectively added with an colorant (or alternatively the instant release layer 10 and/or delayed release layer 20 being added with the colorant) to provide a coloring effect when contacting liquid in order to enhance safety of medicine use. The colorant can be identical to what described above and no further discussion will be given.

The feature of the present invention is a combination of an instant release layer 10 and a delayed release layer 20 to provide an effect of improving sleep quality so as to enhance the practicability and improvement of the present invention. Further, with the coloring effect occurring when the colorant contained in the instant release layer 10, the delayed release layer 20, or the coating layer 40 is brought into contact with liquid, the safety of medicine use can be improved to thereby enhance the practicability and improvement of the present invention. Further, by making the double-layered tablet in the form of a tablet, it can be convenient taken thereby enhancing the convenience of the present invention. Further, with an indent line 30 provided on a surface of the double-layered tablet for half breaking of the tablet, a medicine taker can selectively take only half dose thereby providing flexible dosing and thus enhancing the variation of the present invention. As discussed above, the double-layered zaleplon-contained tablet in accordance with the present invention offers practicability, improvement, convenience, and variation.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A double-layered zaleplon-contained tablet, comprising:
an instant release layer, which comprises zaleplon, lactose, maltose, microcrystalline cellulose, sodium lauryl sulfate, and at least one excipient; and
a delayed release layer, which comprises zaleplon, maltose, microcrystalline cellulose, a polymer, and at least one excipient;
wherein a dissolution curve is obtained by using a 0.1 N hydrochloric acid buffer and pH6.8 phosphoric acid buffer contained in stirring devices having rotatable blades with stirring speeds of 50-200RPM, and the dissolution curve includes an instant release phase and a delayed release phase, in which the instant release phase has a maximum duration of 30 minutes, the delayed release phase represents the remaining dissolution occurring after the instant release phase, a portion of 30-60% of a total amount of zaleplon being released in the instant release phase, and a portion of 90% of the total amount of zaleplon being released in a period of 4-8 hours;
as such, the release of zaleplon is controlled in such a way to follow the dissolution curve.

2. The double-layered zaleplon-contained tablet as claimed in Claim **1,** wherein the polymer of the delayed release layer is selected from a group consisting of hydroxypropyl methyl cellulose, hydroxymethyl cellulose, hydroxyethyl cellulose, hydroxypropyl cellulose, carboxymethyl cellulose, carboxymethylcellulose sodium, methylcellulose, ethylcellulose, gum Arabic, tragacanth, locust bean gum, guar, karaya, gelatin, pectin, casein, and polyethylene glycol.

3. The double-layered zaleplon-contained tablet as claimed in Claim **1,** wherein the excipient comprises one of a disintegrant, a glidant, and a lubricant.

4. The double-layered zaleplon-contained tablet as claimed in Claim **3,** wherein the disintegrant is selected from a group consisting of crospovidone, sodium starch glycolate, cross-carmellose sodium, low-substituted hydroxypropyl cellulose, microcrystalline cellulose, sodium lauryl sulfate, starch, pre-gelatinized starch, hydroxypropy starch, gum Arabic, tragacanth, karaya, pectin, agar, and locust bean gum.

5. The double-layered zaleplon-contained tablet as claimed in Claim **3,** wherein the glidant is selected from a group consisting of silicon dioxide, talc, and cornstarch.

6. The double-layered zaleplon-contained tablet as claimed in Claim **3,** wherein the lubricant is selected from a group consisting of magnesium stearate, calcium stearate, zinc stearate, stearic acid, hydrogenated vegetable oil, mineral oil, talc, and polyethylene glycol.

7. The double-layered zaleplon-contained tablet as claimed in Claim **1,** wherein the instant release layer comprises a colorant for identification purposes, which is selected from a group consisting of curcumins, riboflavins, tartrazine, quinoline yellow, sunset yellow FCF, allura red AC, patent blue V, carmines, azorubine, amaranth, erythrosine, indigotine, chlorophylls, green S, caramels, brown FK, brown HT carotenes, lycopene, luteins, anthocyanidins, gardenia yellow, calcium carbonates, titanium dioxide, iron oxides, aluminum, silver, and gold.

8. The double-layered zaleplon-contained tablet as claimed in Claim **1,** wherein the delayed release layer comprises a colorant for identification purposes, which is selected from a group consisting of curcumins, riboflavins, tartrazine, quinoline yellow, sunset yellow FCF, allura red AC, patent blue V, carmines, azorubine, amaranth, erythrosine, indigotine, chlorophylls, green S, caramels, brown FK, brown HT carotenes, lycopene, luteins, anthocyanidins, gardenia yellow, calcium carbonates, titanium dioxide, iron oxides, aluminum, silver, and gold.

9. The double-layered zaleplon-contained tablet as claimed in Claim **1,** wherein the delayed release layer comprises a medically acceptable organic acid, which is selected from a group consisting of lactic acid, citric acid, malic acid, maleic acid, fumaric acid, tartaric acid, succinic acid, and adipic acid.

10. The double-layered zaleplon-contained tablet as claimed in Claim **1** further comprises a coating layer covering the double-layered tablet formed by combining the instant release layer and the delayed release layer, the coating layer comprising hydroxypropyl methyl cellulose, talc, and polyethylene glycol.

11. The double-layered zaleplon-contained tablet as claimed in Claim **10,** wherein the coating layer comprises an colorant for identification purposes, which is selected from a group consisting of curcumins, riboflavins, tartrazine, quinoline yellow, sunset yellow FCF, allura red AC, patent blue V, carmines, azorubine, amaranth, erythrosine, indigotine, chlorophylls, green S, caramels, brown FK, brown HT carotenes, lycopene, luteins, anthocyanidins, gardenia yellow, calcium carbonates, titanium dioxide, iron oxides, aluminum, silver, and gold.

12. The double-layered zaleplon-contained tablet as claimed in Claim **10,** wherein the coating layer is made of a material selected from a group consisting of Eudragit and Opadry.

13. The double-layered zaleplon-contained tablet as claimed in Claim **1,** wherein the tablet has a surface in which an indent line is formed.
